# EUROPEAN PATENT APPLICATION

(11) **EP 4 455 138 A1**
(43) Date of publication of application: **30.10.2024**
(21) Application number: 22909829.8
(22) Date of filing: 14.12.2022
(51) Int. Cl.: C07D 403/10, C07D 257/04

(54) **METHOD FOR PREPARING HIGH-PURITY LOSARTAN**

(30) Priority: 22.12.2021 CN 202111577892
(71) Applicant: Zhejiang Huahai Pharmaceutical Co., Ltd., Taizhou, Zhejiang 317024 (CN)
(72) Inventor: YANG, Xian, Linhai Taizhou, Zhejiang 317024 (CN); ZHENG, Yang, Linhai Taizhou, Zhejiang 317024 (CN); LI, Aixing, Linhai Taizhou, Zhejiang 317024 (CN); LIU, Jing, Linhai Taizhou, Zhejiang 317024 (CN); LIANG, Zunjun, Linhai Taizhou, Zhejiang 317024 (CN); YAN, Fengfeng, Linhai Taizhou, Zhejiang 317024 (CN)
(74) Representative: Pfenning, Meinig & Partner mbB
(86) International application number: PCT/CN2022/138852
(87) International publication number: WO 2023/116515

(57) **Abstract**

Disclosed in the present invention is a method for preparing high-purity losartan, which belongs to the fields of medicine and chemical engineering. The losartan is obtained by means of a multi-stage temperature reaction in the presence of a catalyst and sodium azide, wherein the temperature in the first stage of the multi-stage temperature reaction is higher than that in the second stage. According to the method, the generation of dimer impurities can be reduced, and the risk of impurities being introduced into a finished losartan potassium product is reduced. (I, II)

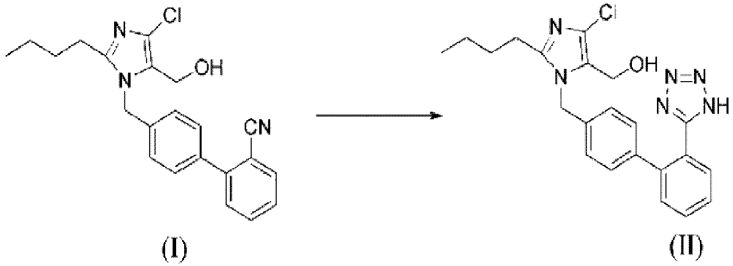

## Description

This application claims the priority of the Chinese Patent Application with the application number of CN202111577892.8, titled "METHOD FOR PREPARING HIGH-PURITY LOSARTAN", filed before the China National Intellectual Property Administration on December 22, 2021, which is incorporated herein by reference in its entirety.

### FIELD OF THE INVENTION

The present invention relates to a preparation method of high-purity losartan, and belongs to the field of medicine and chemical industry.

### BACKGROUND OF THE INVENTION

Losartan Potassium (trade name: Cozaar) is a drug developed by Dupont-Merck in the United States for the treatment of essential hypertension, which is suitable for patients treated with combined medication. Losartan Potassium belongs to Angiotensin II Antagonists, and has a chemical name of 2-butyl-4-chloro-1-[[2'-(1H-tetrazol-5-yl)[1,1'-biphenyl]-4-yl]methyl]-1H-imidazol-5-methanol, monopotassium salt, and the structure is represented as follows:

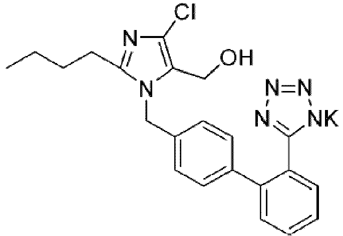

A common synthetic route of losartan reported in literatures is as follows:

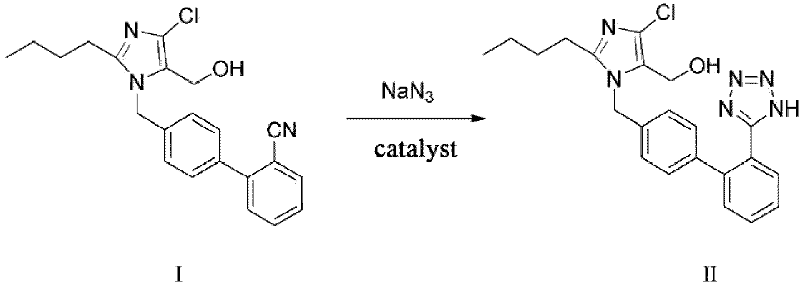

The catalysts used to synthesize losartan are generally ammonium salts, zinc salts or triethylamine salts, and the purity of the obtained losartan is generally 96-97%. In the synthesis process, due to the influence of reaction conditions, dimer impurities as shown in formulae III and IV are easily generated in the reaction, and the obtained losartan has a yellow to brownish yellow appearance, and several decolorization steps are required to obtain qualified losartan potassium. The structural formulae of dimer L (III) and dimer M (IV) are shown below:

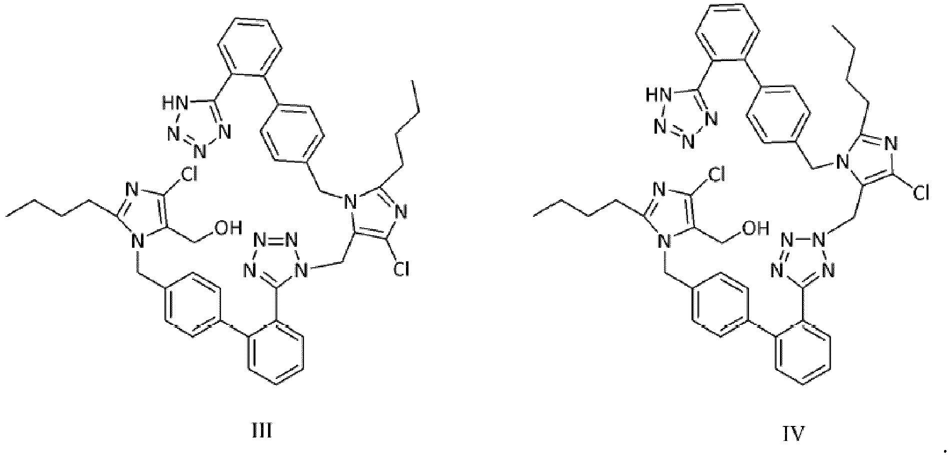

### SUMMARY OF THE INVENTION

The present invention aims to provide a preparation method of losartan with a high yield and a high purity, which can reduce dimer impurities generated during the reaction and improve the appearance of losartan.

In one aspect, the present invention provides a method for preparing losartan, wherein, in the presence of a catalyst and sodium azide, compound I is subjected to a multi-temperature-stage reaction to obtain losartan,

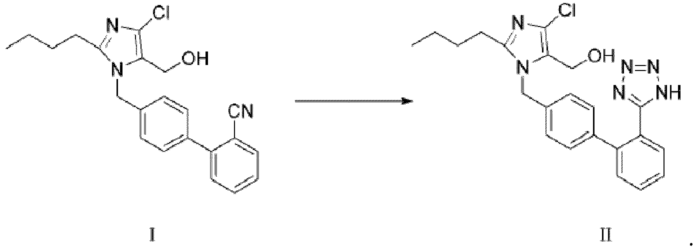

In some embodiments of the present invention, the multi-temperature-stage reaction is a two-temperature-stage reaction between 70°C and 110°C.

In some embodiments of the present invention, the temperature of a first stage of the two-temperature-stage reaction is 90°C to 110°C, the time period of the first stage is 20 h to 30 h, the temperature of a second stage is 75°C to 85°C, and the time period of the second stage is 40 h to 60 h.

In some embodiments of the present invention, the temperature of the first stage of the two-temperature-stage reaction is 90°C to 100°C or 90°C to 95°C.

In some embodiments of the present invention, the temperature of the second stage of the two-temperature-stage reaction is 72°C to 83°C or 83°C to 85°C.

In some embodiments of the present invention, the catalyst is Lewis acid, a strong acid salt of a weak base, or a mixed system of a weak base and a strong acid, and the molar ratio of the compound I to the catalyst is 1:1.5 to 1:3, preferably 1:1.8 to 1:2.2.

In some embodiments of the present invention, the Lewis acid can be zinc chloride or lithium chloride, preferably zinc chloride; the strong acid salt of a weak base is triethylamine hydrochloride, pyridine hydrochloride, triethylamine sulfate or pyridine sulfate, preferably triethylamine hydrochloride; the weak base in the mixed system of weak base and strong acid is preferably triethylamine or tetrahydropyridine, and the strong acid is preferably hydrochloric acid or sulfuric acid, and the molar ratio of the weak base to the strong acid is 1:1 to 2:1.

In some embodiments of the present invention, the reaction is carried out in an organic solvent.

In some embodiments of the present invention, the organic solvent is an aromatic solvent, preferably toluene or xylene.

In some embodiments of the present invention, the ratio of the volume of the organic solvent to the mass of the compound I is 1 mL/g to 1.5 mL/g.

In some embodiments of the present invention, a phase transfer catalyst is also used into the reaction. The phase transfer catalyst is quaternary ammonium salts phase transfer catalyst, preferably benzyltriethylammonium chloride, tetrabutylammonium bromide, tetrabutylammonium chloride, or tetrabutylammonium hydrogen sulfate, and more preferably tetrabutylammonium bromide.

In some embodiments of the present invention, the molar ratio of the phase transfer catalyst to the compound I is 0.01:1 to 0.5:1, preferably 0.01:1 to 0.04:1.

In some embodiments of the present invention, the molar ratio of the input amount of the compound I to sodium azide is 1:2.0 to 1:4, preferably 1:2.3 to 1:3.3.

In some embodiments of the present invention, when the strong acid is added into the system, the temperature of the system is controlled in the range of 10°C to 50°C.

In some embodiments of the present invention, a post-reaction treatment comprises the steps of: adding an alkaline solution for washing, dividing the reaction system into three layers, and separating the material layers.

In some embodiments of the present invention, the alkaline solution is an aqueous sodium carbonate solution or a mixed aqueous solution of sodium chloride and sodium carbonate, and the washing is performed for 1 to 5 times.

In another aspect, the present invention provides a losartan composition, wherein in the losartan, the content of dimer L shown in formula III is less than 0.2%, and the content of dimer M shown in formula IV is less than 0.2%,

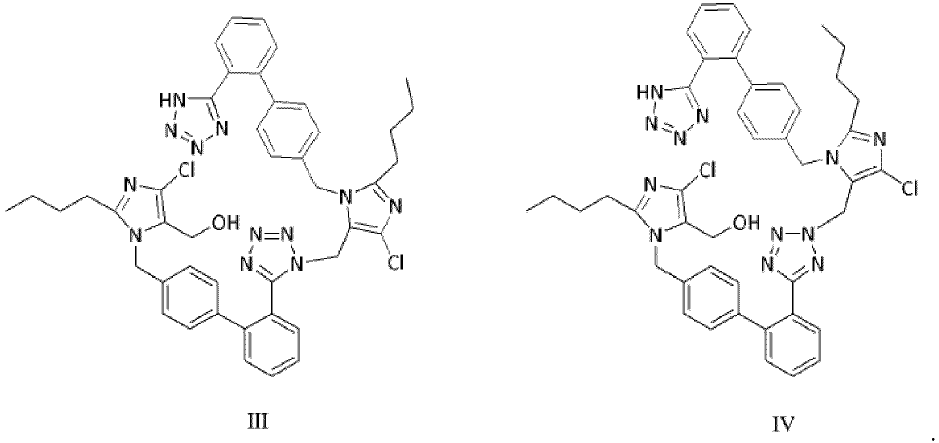

In the losartan prepared by the present invention, the content of dimer L is less than 0.2%, and the content of dimer M is less than 0.2%.

The raw material compound I used in the present invention can be prepared by referring to the patent document WO2007133040A1.

The method of the present invention can improve the quality and appearance of losartan, increase the yield of losartan, reduce the generation of dimer L and dimer M, and reduce the risk of introducing impurities into the finished product of losartan potassium. Therefore, the preparation method of losartan has good practical values.

### DETAILED DESCRIPTION OF THE INVENTION

Unless the contrary is stated in the context, the terms in this present invention have the following meanings.

The terms "having", "including" and "comprising" should be interpreted as open-ended, indicating the existence of the enumerated element(s) but not excluding the existence, appearance or addition of any other one or more elements not enumerated.

All ranges recited herein include those endpoints that recite ranges between two values, unless otherwise stated. Whether indicated or not, all values listed herein include the expected experimental error, technical error and instrument error of the given technology used to measure the value.

In the present invention, unless otherwise specified, % is a weight/weight (w/w) percentage.

Unless otherwise specified, any numerical value, such as the concentration or concentration range mentioned in the present invention, should be understood as being modified in all cases by the term "about".

In the present invention, unless otherwise specified, the term "about" is intended to define its modified numerical value, indicating that such value can vary within a certain range. When there is no recited range (such as the error range or the standard deviation of the average value given in the chart or data table), the term "about" should be understood to mean a larger range including the recited value, as well as a range that is rounded to that number considering significant figures, and a range including plus or minus 5% of the recited value.

In the present invention, the multi-temperature-stage reaction refers to a reaction that is performed for a certain time in different temperature stages, respectively, which is different from naturally heating or cooling. The temperature of each stage is controlled by reaction equipment, and after heating to a specific temperature stage, the reaction is performed while maintaining the temperature. The reaction time of each temperature stage is not less than 1 hour, and the temperature fluctuation range of a specific temperature stage is not more than 1-3 degrees.

For example, a two-temperature-stage reaction refers to a reaction that is performed at a first temperature stage for a first time period and at a second temperature stage for a second time period. In the present invention, the multi-temperature-stage reaction may comprise two, three, four or more stages. The reaction is firstly carried out in a high temperature stage, and then the reaction is carried out at gradually reduced temperature stages. The number of stages of multi-temperature-stage reaction and the reaction time of each temperature stage are adjusted according to the conditions of experiment or production. The reaction progress can be monitored by TLC and HPLC.

In the process of preparing compound II by the reaction of compound I and sodium azide, the compound II may, after being generated, undergo a further dimerization to generate a dimer III or IV, as shown in the following formulae. The amount of dimer impurities produced increases with the increase of the concentration of compound II in the reaction system. The inventors found that when the concentration of compound II is high or the reaction temperature is high, dimer impurities are easily generated, the color of the reaction system is relatively dark, and the obtained losartan product has a brown appearance. Inventor found that the multi-temperature-stage reaction can not only ensure the occurrence of main reaction but also reduce the occurrence of side reactions, thereby reducing the generation of dimer impurities, resulting in a product having a high-purity and a good appearance.

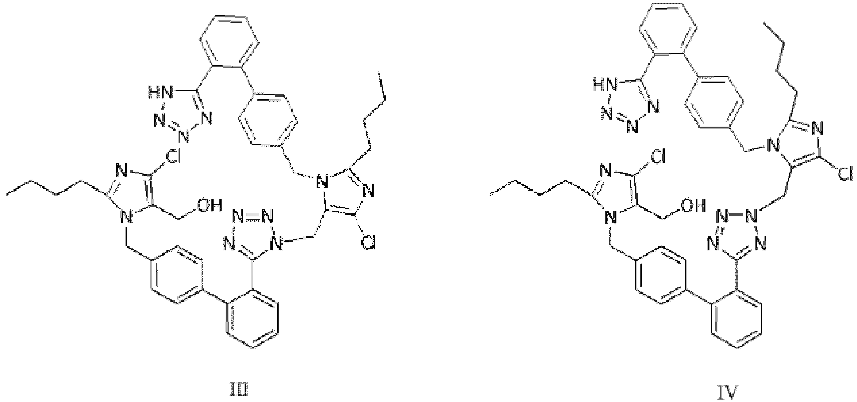

In the present invention, the main reaction refers to the reaction that produces compound II, and the side reactions refer to the reactions that produce dimer impurities.

In some embodiments of the present invention, the two-temperature-stage reaction is performed between 70°C and 110°C.

In some embodiments of the present invention, the temperature of the first stage of the multi-temperature-stage reaction is 90°C to 110°C (for example, 90°C, 95°C, 100°C, 105°C, 110°C or any values or ranges there between), and the time period of the first stage is 20 h to 30 h (for example, 20 h, 22 h, 23 h, 24 h, 25 h, 26 h, 28 h, 30 h or any values or ranges there between); the temperature of the second stage is 70°C to 85°C (for example, 75°C, 80°C, 85°C or any values or ranges there between), and the time period of the second stage is 40 h to 60 h (for example, 40 h, 45 h, 50 h, 55 h, 60 h or any values or ranges there between).

In some embodiments of the present invention, the temperature of the first stage of the two-temperature-stage reaction is 90°C to 100°C.

In some embodiments of the present invention, the temperature of the first stage of the two-temperature-stage reaction is 90°C to 95°C (for example, 90°C, 91°C, 92°C, 93°C, 94°C, 95°C or any values or ranges there between).

In some embodiments of the present invention, the temperature of the second stage of the two-temperature-stage reaction is 72°C to 83°C (for example, 72°C, 73°C, 74°C, 75°C, 76°C, 77°C, 78°C, 79°C, 80°C, 81°C, 82°C, 83°C or any values or ranges there between).

In some embodiments of the present invention, the temperature of the second stage of the two-temperature-stage reaction is 83°C to 85°C (for example, 83°C, 84°C, 85°C or any values or ranges there between).

In some embodiments of the present invention, the time period of the first stage of the two-temperature-stage reaction is 22 h to 28 h or 22 h to 25 h.

In some embodiments of the present invention, the time period of the second stage of the two-temperature-stage reaction is 45 h to 55 h or 45 h to 50 h.

The "catalyst" in the present invention can be used as a proton donor in the reaction system.

The catalyst of the present invention can be Lewis acid, a strong acid salt of a weak base, or a mixed system of weak base and strong acid.

Lewis acid of the present invention includes, but is not limited to, zinc chloride or lithium chloride.

In some embodiments of the present invention, the Lewis acid of the present invention is zinc chloride.

The strong acid salt of weak base of the present invention includes, but is not limited to, triethylamine hydrochloride, pyridine hydrochloride, triethylamine sulfate, and pyridine sulfate.

In some embodiments of the present invention, the strong acid salt of weak base of the present invention is triethylamine hydrochloride.

In some embodiments of the present invention, the Lewis acid is triethylamine sulfate.

The weak base in the mixed system of weak base and strong acid of the present invention includes, but is not limited to, triethylamine and tetrahydropyridine, and the strong acid includes, but is not limited to, hydrochloric acid and sulfuric acid; and the molar equivalent ratio of the weak base to the strong acid is about 1:1 to 3:1.

In some embodiments of the present invention, the mixed system of weak base and strong acid is a mixed system of triethylamine and concentrated hydrochloric acid, wherein the molar ratio of triethylamine to hydrochloric acid can be 1:1 to 2:1, preferably 1:1 to 1.2:1, e.g., 1:1, 1.1:1, 1.12:1, 1.14:1, 1.16:1, 1.18:1, 2:1 or any values or ranges there between.

In some embodiments of the present invention, the mixed system of weak base and strong acid is a mixed system of triethylamine and concentrated sulfuric acid, wherein the molar ratio of triethylamine to concentrated sulfuric acid can be 1.5:1 to 2.5:1, such as 1.5:1, 1.7:1, 1.9:1, 2.0:1, 2.1:1, 2.2:1, 2.3:1, 2.5:1 or any values or ranges there between.

In some embodiments of the present invention, the molar ratio of the compound I to the catalyst is 1:1.5 to 1:3, such as 1:1.5, 1:2, 1:2.5, 1:3 or any values and ranges there between.

In some embodiments of the present invention, the molar ratio of the compound I to the catalyst is 1:1.8 to 1:2.2, such as 1:1.8, 1:1.9, 1:2.0, 1:2.1, 1:2.2 or any values and ranges there between.

In some embodiments of the present invention, the reaction is carried out in an organic solvent.

In some embodiments of the present invention, the organic solvent is an aromatic solvent.

In some embodiments of the present invention, the organic solvent is toluene or xylene.

In some embodiments of the present invention, the volume-to-mass ratio of the organic solvent to the compound I is 1-1.5 mL/g, such as 1 mL/g, 1.1 mL/g, 1.2 mL/g, 1.3 mL/g, 1.4 mL/g or 1.5 ml/g.

The term "phase transfer catalyst" used in the present invention refers to a kind of catalyst that can help the reactants to transferring from one phase to another phase where the reaction can occur, thus accelerating the reaction rate of heterogeneous system. The phase transfer catalyst in the present invention can be ammonium salts phase transfer catalyst, preferably benzyltriethylammonium chloride, tetrabutylammonium bromide, tetrabutylammonium chloride or tetrabutylammonium hydrogen sulfate, and more preferably tetrabutylammonium bromide.

In some embodiments of the present invention, the phase transfer catalyst is tetrabutylammonium bromide, and the molar ratio of tetrabutylammonium bromide to compound I is 0.01:1 to 0.5:1, such as 0.01:1, 0.05:1, 0.1:1, 0.2:1, 0.3:1, 0.4:1, 0.5:1 or any values and ranges there between.

In some embodiments of the present invention, the molar ratio of tetrabutylammonium bromide to the compound I is 0.01 to 0.04:1, such as 0.01:1, 0.02:1, 0.03:1, 0.04:1 or any values and ranges there between.

In some embodiments of the present invention, the molar ratio of the input amount of compound I to sodium azide is 1:2.0 to 1:4, preferably 1:2.3 to 1:3.3, such as 1:2.3, 1:2.4, 1:2.5, 1:2.6, 1:2.7, 1:2.8, 1:2.9, 1:3.0, 1:3.1, 1:3.2, 1:3.3 or any values and ranges there between.

In the present invention, heat may be released when the strong acid is added, and the temperature of the system needs to be controlled.

In some embodiments of the present invention, when strong acid is added, the temperature of the system is controlled within the scope between 10°C and 50°C.

In the present invention, the post-treatment step after the reaction is not particularly limited, and may involve a conventional post-treatment method in the field.

In some embodiments of the present invention, the post-treatment step after the reaction comprises: adding an alkaline solution for washing, dividing the reaction system into three layers, and separating a material layer.

The term "alkaline solution" in the present invention refers to an aqueous solution with a pH greater than 7, such as 8, 9, 10, 11, 12 or 13. Exemplary bases for preparing an "alkaline solution" include, but are not limited to, hydroxide salts (such as sodium hydroxide, potassium hydroxide and lithium hydroxide), carbonates (such as sodium carbonate, potassium carbonate, lithium carbonate and calcium carbonate) or bicarbonates (such as sodium bicarbonate and potassium bicarbonate).

In some embodiments of the present invention, the alkaline solution is an aqueous sodium carbonate solution or a mixed aqueous solution of sodium chloride and sodium carbonate, and the washing is performed for 1 to 5 times.

In some embodiments of the present invention, the post-treatment step includes: after the reaction is completed, cooling the temperature of materials to 50°C to 60°C, adding aqueous sodium carbonate solution, stirring the solution and standing for layering; (b) adding a mixed aqueous solution of sodium chloride and sodium carbonate into a material layer for washing; (c) acidifying, filtering, washing and drying the material layer to obtain losartan.

In some embodiments of the present invention, the step (b) is repeated for 1-3 times, such as 1, 2 or 3 times.

In some embodiments of the present invention, the post-treatment step includes: after the reaction is completed, cooling the temperature of the reaction mixture, and then adding sodium hydroxide solution and water into the mixture for saponification; standing for liquid separation, adding sodium nitrite and water into a water phase, followed by stirring; cooling, and acidifying the solution by adding hydrochloric acid, and then adding ethyl acetate for dispersion, and stirring the mixture for crystallization; suction filtering the solution, and drying the resultant to obtain losartan.

In the present invention, the term "concentrated hydrochloric acid" refers to an aqueous solution of hydrogen chloride with a mass fraction of about 37% (for example, 36%, 37%, 38% or any values or ranges there between), with a density of about 1.1492 g/ml.

In the present invention, triethyl amine and triethylamine represent the same meaning.

The embodiments of the present invention will be further described in detail by referring to the following examples, but the present invention is not limited to the following examples.

Unless otherwise specified, the reagents used in this present invention are all commercially available, in which the concentration of concentrated hydrochloric acid is an aqueous hydrogen chloride solution with a mass fraction of 36% and the concentrated sulfuric acid is a 98 wt.% sulfuric acid solution.

### Example 1

27.2 g of tetrahydropyridine and 90 mL of toluene were sequentially added into a four-necked flask, and 19.0 g of concentrated sulfuric acid was added dropwise under stirring. After the dropwise addition, 66.0 g of compound I, 26.0 g of sodium azide and 0.8 g of tetrabutylammonium bromide were added into the system sequentially, and the mixture was stirred for 25 hours under a reaction temperature of 95°C, which was then cooled to 80°C and stirred for 50 hours while maintaining the temperature. After the reaction is completed, the solution was extracted, acidified, filtered, washed and dried to obtain 67.3 g of losartan having a light yellow appearance, with a yield of 91.6% and a purity of 99.1%, wherein the content of dimer L is 0.10%, and the content of dimer M is 0.13%.

### Example 2

39.2 g of Triethylamine and 90 mL of toluene were sequentially added into a four-necked flask, and 19.0 g of concentrated sulfuric acid was added dropwise under stirring. After the dropwise addition, 66.0 g of compound I, 26.0 g of sodium azide and 0.8 g of tetrabutylammonium bromide were added into the system sequentially, and the mixture was stirred for 50 hours under a reaction temperature of 95°C, which was then then cooled to 80°C and stirred for 50 hours while maintaining the temperature. After the reaction is completed, the solution was extracted, acidified, filtered, washed and dried to obtain 67.5g of losartan having a light yellow appearance, with a yield of 91.8% and a purity of 99.2%, wherein the content of dimer L is 0.08%, and the content of dimer M is 0.10%.

### Example 3

1120 L of toluene and 500 kg of triethylamine were put into a reaction kettle, and 250 kg of concentrated sulfuric acid was added dropwise while maintaining the temperature between 10°C and 50°C. 900 kg of compound I and 30 kg of tetrabutylammonium bromide were added, followed by the addition of 500 kg of sodium azide. The mixture was stirred for 20 to 30 hours while maintaining the temperature between 90°C and 110°C. Then the mixture was cooled to 75°C to 85°C and stirred further for 40 to 60 hours. The material was cooled to a temperature of 50°C to 60°C, and then transferred to a saponification kettle. 4000 L of aqueous sodium carbonate solution was added, stirred, and allowed to stand, such that the reaction system is divided into three layers, wherein an upper layer is a water-insoluble organic solvent layer, a middle layer is a material layer, and a lower layer is a water layer. 2000 L of a mixed aqueous solution of sodium chloride and sodium carbonate was added to wash the material layer, and then the material layer was acidified, filtered, washed and dried to obtain 940 kg of losartan having an off-white appearance, with a yield of 93.8% and a purity of 99.2%, wherein the content of dimer L is 0.07%, and the content of dimer M is 0.09%.

### Example 4

1120 L of xylene and 500 kg of triethylamine were put into a reaction kettle, and 140 L of concentrated hydrochloric acid was added dropwise while maintaining the temperature between 10°C to 50°C. 900 kg of compound I and 30 kg of tetrabutylammonium bromide were added; followed by the addition of 500 kg of sodium azide. The mixture was stirred for 20 to 30 hours while maintaining the temperature between 90°C to 110°C. Then the mixture was cooled to 75°C to 85°C and stirred for 40 to 60 hours. The material was cooled to a temperature of 50°C to 60°C, and then transferred to a saponification kettle, and 4000 L of aqueous sodium carbonate solution was added, stirred, and allowed to stand, such that the reaction system is divided into three layers, wherein an upper layer is a water-insoluble organic solvent layer, a middle layer is a material layer, and a lower layer is a water layer. 2000 L of a mixed aqueous solution of sodium chloride and sodium carbonate was added to wash the material layer, and then the material layer was acidified, filtered, washed and dried to obtain 931 kg of losartan having an off-white appearance, with a yield of 92.9% and a purity of 99.3%, wherein the content of dimer L is 0.06%, and the content of dimer M is 0.07%.

### Comparative Example 1

27.2 g of tetrahydropyridine and 160 mL of toluene were sequentially added into a four-necked flask, and then 19.0 g of concentrated sulfuric acid was added dropwise under stirring. After the dropwise addition, 66.0 g of compound I, 26.0 g of sodium azide and 0.8 g of tetrabutylammonium bromide were added into the system successively, and the temperature was raised to 95°C and reaction was carried out for 42 h. After the reaction is completed, the resultant was extracted, acidified, filtered, washed and dried to obtain 62.5 g of losartan having a brown appearance, with a yield of 85.0% and a purity of 98.5%, wherein the content of dimer L is 0.41%, and the content of dimer M is 0.46%.

### Comparative Example 2

39.2 g of triethylamine and 330 mL of toluene were sequentially added into a four-necked flask, and 19.0 g of concentrated sulfuric acid was added dropwise under stirring. After the dropwise addition, 66.0 g of compound I, 36.7 g of sodium azide and 2.2 g of tetrabutylammonium bromide were added into the system successively, and the mixture was heated to 95°C and stirred for 50 hours. After the reaction is completed, 300 mL of aqueous sodium carbonate solution was added, and the mixture was stirred and standing, such that the reaction system was divided into three layers, wherein an upper layer is a water-insoluble organic solvent layer, a middle layer is a material layer, and a lower layer is a water layer. 150 mL of a mixed aqueous solution of sodium chloride and sodium carbonate was added to wash the material layer, and then the material layer was acidified, filtered, washed and dried to obtain 60.2 g of losartan having a brown appearance, with a yield of 81.9% and a purity of 98.5%, wherein the content of dimer L is 0.43%, and the content of dimer M is 0.50%.

### Comparative Example 3

4500 L of toluene and 500 kg of triethylamine were put into a reaction kettle, and 250 kg of concentrated sulfuric acid was added dropwise while maintaining the temperature between 10°C and 50°C. 900 kg of Compound I and 30 kg of tetrabutylammonium bromide were added, followed by the addition of 500 kg of sodium azide. The mixture was stirred for 50 hours while maintaining the temperature at 95°C. Then the material was cooled to a temperature of 50°C to 60°C, and was transferred to a saponification kettle, and 4000 L of aqueous sodium carbonate solution was added, stirred, and allowed to stand, such that the reaction system is divided into three layers, wherein an upper layer is a water-insoluble organic solvent layer, a middle layer is a material layer, and a lower layer is a water layer. 2000 L of a mixed aqueous solution of sodium chloride and sodium carbonate was added to wash the material layer, and then the material layer was acidified, filtered, washed and dried to obtain 831 kg of losartan having a brown appearance, with a yield of 82.9% and a purity of 98.4%, wherein the content of dimer L is 0.44%, and the content of dimer M is 0.49%.

All documents mentioned in the present invention are incorporated by reference in this application as if each document were individually incorporated by reference. In addition, it should be understood that after reading the above teachings of the present invention, those skilled in the art can make various changes or modifications to the present invention, and these equivalent forms also fall within the scope of the appended claims.

## Claims

1. A method for preparing losartan, comprising subjecting compound I to a multi-temperature-stage reaction in the presence of a catalyst and sodium azide to obtain losartan, wherein the temperature of a first stage of the multi-temperature-stage reaction is higher than that of a second stage,

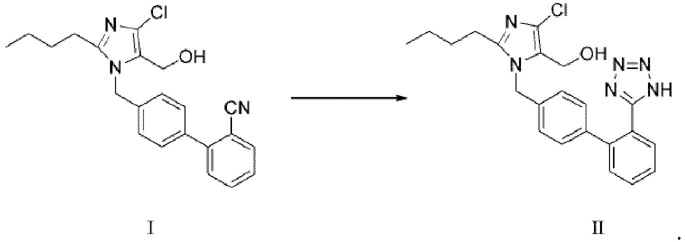

2. The method according to claim 1, wherein the multi-temperature-stage reaction is a two-temperature-stage reaction between 70°C and 110°C.

3. The method according to claim 2, wherein the temperature of the first stage of the two-temperature-stage reaction is 90°C to 110°C, the time period of the first stage is 20 hours to 30 hours, the temperature of the second stage is 75°C to 85°C, and the time period of the second stage is 40 hours to 60 hours.

4. The method according to claim 2, wherein the temperature of the first stage of the two-temperature-stage reaction is 90°C to 100°C or 90°C to 95°C.

5. The method according to claim 2, wherein the temperature of the second stage of the two-temperature-stage reaction is 72°C to 83°C or 83°C to 85°C.

6. The method according to claim 1, wherein the catalyst is Lewis acid, a strong acid salt of a weak base, or a mixed system of weak base and strong acid, and the molar ratio of the compound I to the catalyst is 1:1.5 to 1:3, preferably 1:1.8 to 1:2.2.

7. The method according to claim 6, wherein the Lewis acid can be zinc chloride or lithium chloride, preferably zinc chloride; the strong acid salt of a weak base is triethylamine hydrochloride, pyridine hydrochloride, triethylamine sulfate or pyridine sulfate, preferably triethylamine hydrochloride; the weak base in the mixed system of weak base and strong acid is preferably triethylamine or tetrahydropyridine, and the strong acid is preferably hydrochloric acid or sulfuric acid, and the molar ratio of the weak base to the strong acid is 1:1 to 2:1.

8. The method according to claim 1, wherein the reaction is carried out in an organic solvent.

9. The method according to claim 1, wherein the organic solvent is an aromatic solvent, preferably toluene or xylene.

10. The method according to claim 9, wherein the ratio of the volume of the organic solvent to the mass of the compound I is 1 mL/g to 1.5 mL/g.

11. The method according to claim 1, wherein the reaction also uses a phase transfer catalyst, wherein the phase transfer catalyst is a quaternary ammonium salt phase transfer catalyst, preferably benzyl triethylammonium chloride, tetrabutylammonium bromide, tetrabutylammonium chloride, or tetrabutylammonium hydrogen sulfate, and more preferably tetrabutylammonium bromide.

12. The method according to claim 11, wherein the molar ratio of the phase transfer catalyst to the compound I is 0.01:1 to 0.5:1, preferably 0.01:1 to 0.04:1.

13. The method according to claim 1, wherein the molar ratio of the compound I to sodium azide is 1:2.0 to 1:4, preferably 1:2.3 to 1:3.3.

14. The method according to claim 7, wherein when the strong acid is added into the system, and the temperature of the system is controlled between 10°C and 50°C.

15. The method according to claim 1, wherein a post-reaction treatment comprises the steps of: adding an alkaline solution for washing, dividing the reaction system into three layers, and separating a material layer.

16. The method according to claim 15, wherein the alkaline solution is an aqueous sodium carbonate solution or a mixed aqueous solution of sodium chloride and sodium carbonate, and the washing is performed for 1 to 5 times.

17. A losartan composition, wherein the content of dimer L represented by formula III is less than 0.2%, and the content of dimer M represented by formula IV is less than 0.2%,
